# EUROPEAN PATENT APPLICATION

(11) **EP 1 788 094 A1**
(43) Date of publication of application: **23.05.2007**
(21) Application number: 05780878.4
(22) Date of filing: 23.08.2005
(51) Int. Cl.: C12Q 1/68, C12N 15/09, C12Q 1/02, G01N 33/15, G01N 33/48, G01N 33/50, G01N 33/53, G01N 33/566, C07K 16/18

(54) **METHOD OF DISTINGUISHING SPINAL CORD NEURON TYPE TARGETING CORL1 GENE**

(30) Priority: 24.08.2004 JP 2004243588
(71) Applicant: Eisai R&D Management Co., Ltd., Tokyo 112-8088 (JP)
(72) Inventor: Ono, Yuichi, c/o KAN Research Institute, Inc., Shimogyo-ku, Kyoto-shi, Kyoto 6008815 (JP); Nakagawa, Yasuko, c/o KAN Research Institute, Inc., Shimogyo-ku, Kyoto-shi, Kyoto 6008815 (JP); Nakatani, Tomoya, c/o KAN Research Institute, Inc., Shimogyo-ku, Kyoto-shi, Kyoto 6008815 (JP)
(74) Representative: Vossius & Partner
(86) International application number: PCT/JP2005/015245
(87) International publication number: WO 2006/022243

(57) **Abstract**

As a result of screening for genes that are selectively expressed in fetal mouse brain region by subtraction method, the present inventors obtained a cDNA fragment encoding Corl1. The expression of Corl1 was examined by RT-PCR, *in situ* hybridization, and immunostaining using polyclonal antibodies. The results demonstrated that Corl1 was especially expressed at a high level of selectively in the central nervous system during embryonic stages. The expression patterns of Corl1 determined using various markers in embryonic spinal cord were compared to identify types of neurons expressing Corl1. The results revealed that Corl1 was specifically expressed in spinal cord interneurons dI4, dI5, dILA, and dILB. Accordingly, the present invention provides for discrimination between dI4 and dI6, neurons which previously could only be discriminated based on developmental location, using the expression of Corl1 as an indicator.

## Description

### Technical Field

The present invention relates to the Corl1 gene that is expressed specifically in spinal cord interneurons dI4, dI5, dILA, and dILB and uses of the gene in identifying types of spinal neurons.

### Background Art

The spinal nervous system, a component of the central nervous system, plays an important role in the regulation of motion and sensation. Regeneration-based therapeutic methods have been investigated for use in the treatment of damages to the spinal nervous system, such as spinal cord injury. Such regeneration may be promoted, for example, through the transplantation of spinal neurons differentiated *in vitro* from ES cells or the like, or, regenerated *in vivo* from patient-derived neural stem cells.

A highly efficient method for inducing the differentiation of ES cells to spinal cord motor neurons has been described in the literature (Non-patent Document 1). Furthermore, the isolation of precursor cells of spinal cord motor neurons from ES cells having knockin GFP in the locus of HB9, a motor neuron-specific marker, has also been described (Non-patent Document 1). In addition, recent discoveries have elucidated the details of the mechanisms underlying prenatal development of spinal neurons other than the motor neurons (Non-patent Documents 2 to 5). Based on such findings, it is expected that various spinal neurons can be efficiently prepared from ES cells and neural stem cells.

In the context of regeneration therapy, it is important to identify the details of the cell populations of the transplanted material, both with respect to therapeutic effect and safety. In terms of enhancing the therapeutic effect, it may also be important to induce the *in vitro* and *in vivo* differentiation only for the required neurons. To achieve this goal, it is essential that one be able to identify individual neuron cells in detail.

To date, at least about 15 types of different neurons have been identified in the spinal cord (Non-patent Documents 2 to 5). Various homeobox transcription factors which are selectively expressed in some spinal neuron types have also been identified. Individual spinal neuron types can be identified using combinations of these factors expressed.

However, for some spinal neuron types, markers with specific expression have yet to be identified. While such cells can be identified based on the development location in embryonic development, it can be difficult to identify such spinal neurons in populations that contain a mixture of *in-vitro*-differentiation-induced spinal neurons, populations of *in vivo* spinal neurons that have migrated after development, and populations of spinal neurons that have regenerated in adults.

Prior art literature related to the invention described in the instant application include:
[Non-patent Document 1] Wichterle H, Lieberam I, Porter AP and Jessell TM. Directed differentiation of embryonic stem cells into motor neurons. Cell 2002 Aug; 110:385-397
[Non-patent Document 2] Jessell TM. Neuronal specification in the spinal cord: Inductive signals and transcriptional codes. Nat Rev Genetics 2000 Oct; 1(1):20-29.(Review)
[Non-patent Document 3] Caspary T, Anderson KV. Patterning cell types in the dorsal spinal cord: what the mouse mutants say. Nat Rev Neurosci. 2003 Apr;4(4):289-97.(Review)
[Non-patent Document 4] Muller T, Brohmann H, Pierani A, Heppenstall PA, Lewin GR, Jessell TM, Birchmeier C. The homeodomain factor lbx1 distinguishes two major programs of neuronal differentiation in the dorsal spinal cord. Neuron. 2002 May 16; 34(4):551-62.
[Non-patent Document 5] Gross MK, Dottori M, Goulding M. Lbx1 specifies somatosensory association interneurons in the dorsal spinal cord. Neuron. 2002 May 16;34(4):535-49.

### Disclosure of the Invention

The present invention was achieved in view of such circumstances. One objective of the present invention is to provide methods and reagents for identifying types of spinal neurons. More specifically, an objective of the present invention is to provide methods for identifying the spinal neurons dI1, dI2, dI3, dI4, dI5, dI6, dILA, or dILB using the expression of the endogenous Corl1 (Corepressor for Lbx1) gene as an indicator, and reagents for detecting the expression of the endogenous Cor1 1 gene by such methods.

To achieve the above-described objective, the present inventors screened for genes that were selectively expressed in the brain region of the fetal mouse using the subtraction method. As a result, a cDNA fragment encoding Corl1 was obtained. The expression of Corl1 was investigated by RT-PCR, *in situ* hybridization, and immunostaining using polyclonal antibodies. The results showed that Corl1 was selectively expressed at a high level in the central nervous system at particular fetal stages. Then, the present inventors closely investigated the expression of Corl1 in fetal spinal cord. When compared with various markers to identify the types of Corl1-expressing neurons, it was revealed that Corl1 was specifically expressed in spinal cord interneurons dI4, dI5, dILA, and dILB.

Spinal cord neurons develop during the embryonic stages. The neurons migrate to each destination to construct the ultimate functional tissues. Interneurons that transmit sensation are developed in the dorsal region of the spinal cord, and then ultimately migrate to the region called the "dorsal horn". Different types of such neurons are distinguished by developmental stage and expression of various markers. However, to date, no known marker is able to distinguish between dI4 and dI6. Accordingly, one must analyze the developmental location and direction of migration in order to distinguish between the two. Through discovery of the spinal neuron-specific expression of Corl1, the present invention enables the use of Corl1 as a marker to identify spinal cord interneurons. More specifically, the present invention provides:
[1] a reagent for identifying types of spinal neurons, said reagent comprising as an active component a polynucleotide that hybridizes to a transcript of a Corl1 gene;
[2] a reagent for identifying types of spinal neurons, said reagent comprising as an active component a polynucleotide that hybridizes under stringent conditions to at least one polynucleotide having a nucleotide sequence selected from the group consisting of SEQ ID NOs: 1, 3, and 5;
[3] a reagent for identifying types of spinal neurons, said reagent comprising as an active component an antibody that binds to a translation product of a Corl1 gene;
[4] a reagent for identifying types of spinal neurons, comprising as an active component an antibody that binds to at least one polypeptide having an amino acid sequence selected from the group consisting of SEQ ID NOs: 2, 4, and 6, or a partial sequence thereof;
[5] a reagent of any one of [1] to [4], wherein the target spinal neuron to be identified is dI1, dI2, dI3, dI4, dI5, dI6, dILA, or dILB;
[6] a kit for identifying types of spinal neurons, said kit comprising one or more polynucleotides that hybridize to a transcript of a Corl1 gene in combination with one or more polynucleotides that hybridize to a transcript of one or more genes selected from the group consisting of Brn3a, Pax2, Lbx1, Lim1, Lim2, LH2A, LH2B, Isl1, Lmx1b, Tlx1, and Tlx3;
[7] a kit for identifying types of spinal neurons, said kit comprising as an active components a polynucleotide that hybridizes under stringent conditions to at least one polynucleotide having a nucleotide sequence selected from the group consisting of SEQ ID NOs: 1, 3, and 5 and a polynucleotide that hybridizes under stringent condition to a transcript of at least one gene selected from the group consisting of Brn3a, Pax2, Lbx1, Lim1, Lim2, LH2A, LH2B, Isl1, Lmx1b, Tlx1, and Tlx3;
[8] a kit for identifying types of spinal neurons, said kit comprising an antibody that binds to a translation product of a Corl1 gene in combination with an antibody that binds to a translation product of one or more genes selected from the group consisting of Brn3a, Pax2, Lbx1, Lim1, Lim2, LH2A, LH2B, Isl1, Lmx1b, Tlx1, and Tlx3;
[9] a kit for identifying types of spinal neurons, said kit comprising as active components an antibody that binds to at least one polypeptide having an amino acid sequence selected from the group comprising SEQ ID NOs: 2, 4, and 6, or a partial sequence thereof and an antibody that binds to a translation product of at least one gene is selected from the group consisting of Brn3a, Pax2, Lbx1, Lim1, Lim2, LH2A, LH2B, Isl1, Lmx1b, Tlx1, and Tlx3;
[10] a kit of any one of [6] to [9], wherein the target spinal neuron to be identified is dI1, dI2, dI3, dI4, dI5, dI6, dILA, or dILB;
[11] a method for identifying types of spinal neurons, said method comprising the step of detecting a transcript or translation product of a Corl1 gene in spinal neurons;
[12] the method of [11], said method comprising the step of detecting a transcript or translation product of one or more genes selected from the group consisting of Brn3a, Pax2, Lbx1, Lim1, Lim2, LH2A, LH2B, Isl1, Lmx1b, Tlx1, and Tlx3;
[13] the method of [11] or [12], wherein the target spinal neuron to be identified is dI1, dI2, dI3, dI4, dI5, dI6, dILA, or dILB;
[14] a method for identifying types of spinal neurons, comprising the steps of contacting spinal neurons with:
   (1) a polynucleotide that hybridizes under stringent conditions to at least one polynucleotide having a nucleotide sequence selected from the group consisting of SEQ ID NOs: 1, 3, and 5; or
   (2) an antibody that binds to a polypeptide comprising an amino acid sequence selected from the group consisting of SEQ ID NOs: 2, 4, and 6, or a partial sequence thereof;
[15] the method of [14], comprising the steps of contacting spinal neurons with:
   (1) a polynucleotide which hybridizes under stringent conditions to a transcript of at least one gene selected from the group consisting of Brn3a, Pax2, Lbx1, Lim1, Lim2, LH2A, LH2B, Isll, Lmx1b, Tlx1, and Tlx3; or
   (2) an antibody that binds to a translation product of at least one gene selected from the group consisting of Brn3a, Pax2, Lbx1, Lim1, Lim2, LH2A, LH2B, Isl1, Lmx1b, Tlx1, and Tlx3;
[16] the method of [14] or [15], further comprising the step of discriminating the group consisting of at least one spinal neuron type selected from dI1, dI2, dI3, and dI6 and the group consisting of at least one spinal neuron type selected from dI4, dI5, dILA, and dILB;
[17] the method of any one of [14] to [16], said method comprising the step of discriminating between a spinal neuron type that expresses a transcript of one or more genes selected from the group consisting of Brn3a, Pax2, Lbx1, Lim1, Lim2, LH2A, LH2B, Isl1, Lmx1b, Tlx1, and Tlx3 and a spinal neuron type that does not express a transcript of one or more genes selected from the group consisting of Brn3a, Pax2, Lbx1, Lim1, Lim2, LH2A, LH2B, Isll, Lmx1b, Tlx1, and Tlx3;
[18] A method for screening for compounds that induce differentiation of cells that have a potential to differentiate into spinal neurons, said method comprising the steps of:
   (a) inducing differentiation of spinal neurons from potential differentiating spinal neurons in the presence of a test sample;
   (b) detecting a transcript or translation product of a Corl1 gene in the differentiated neurons; and
   (c) selecting a test sample that increases the level of the Corl transcript or translation product as compared with the level determined in the absence of the test sample;
[19] the method of [18], wherein the potential spinal nerve differentiating cells are ES cells; and the present invention relates to,
[20] the use of:
   (a) a polynucleotide that hybridizes to a transcript of a Corl1 gene; or
   (b) an antibody which binds to a translation product of a Corl1 gene;
   in the production of reagents for identifying types of spinal neurons.

### Brief Description of the Drawings

Fig. 1 is a schematic diagram showing the structure and homology of Corl1.
Fig. 2 is a photograph showing the expression of Corl1 in tissues of adult mouse, and in the afterbrain and spinal cord of the E 12.5 mouse embryo.
Fig. 3 is a photograph showing the comparison of expression levels of Corl1, Pax7, and βIII-tubulin in E 13.25 mouse embryo spinal cord.
Fig. 4 is a photograph showing the comparison of expression levels of Corl1, Brn3a, Lim1 and Isl1 in Corl1 E10.75 mouse embryo spinal cord.
Fig. 5 is a photograph showing the comparison of expression levels of Corl1, Brn3a, and Lim 1 in Corl1 E13.25 mouse embryo spinal cord.
Fig. 6 is a schematic diagram showing the expression pattern of Corl1 in spinal cord at various developmental stages. "TFs" refers to transcription factors.
Fig. 7 is a photograph showing the expression of Corl1 in spinal neurons that were differentiated from ES cells *in vitro.*
Fig. 8 is a photograph showing the expression of Corl1 in spinal neurons that were differentiated from ES cells *in vitro.*

### Best Mode for Carrying out the Invention

The present invention provides reagents for identifying types of spinal neurons, such reagents including as active component one or more polynucleotides that hybridize to a Corl1 gene transcript. The length of the one or more polynucleotides useful in the context of the present invention is not particularly limited and includes so-called "oligonucleotides".

The present inventors discovered that the Corl1 gene is substantially expressed among spinal neurons, in dI4, dI5, dILA, and dILB, but not substantially expressed in dI1, dI2, dI3, and dI6. Thus, representative spinal neuron types serving as targets to be identified by the reagents of the present invention include dI1, dI2, dI3, dI4, dI5, dI6, dILA, and dILB.

Herein, the phrase "identifying types of spinal neurons" not only refers to target spinal neurons that are identified as specific spinal neuron types but also includes target spinal neurons which are identified as not being cells of specific spinal neuron types. For example, when the Corl1 gene is substantially expressed in the target spinal neurons, the spinal neurons can be identified as "possibly any one of dI4, dI5, dILA, and dILB", or the cells are "not of dI1, dI2, dI3, or dI6". Meanwhile, when the Corl1 gene is substantially not expressed in the target spinal neurons, the spinal neurons can be identified as "possibly any one of dI1, dI2, dI3, or dI6", or the cells "are not of dI4, dI5, dILA, and dILB".

The "Corl1 gene" that is used as an indicator for identifying types of spinal neurons according to the present invention is not particularly limited, as long as it is specifically expressed in spinal neurons as described above. Accordingly, various types of vertebrate Corl1 gene are included in the present invention.

The known nucleotide sequence for the mouse Corl1 gene is set forth in SEQ ID NO: 1; its amino acid sequence is set forth in SEQ ID NO: 2. In the context of the present invention, the Corl1 gene also includes its homologues, for example, human Corl1 (the nucleotide sequence of which is set forth in SEQ ID NO: 3, and the amino acid sequence of which is set forth in SEQ ID NO: 4) and rat Corl1 (the nucleotide sequence of which is set forth in SEQ ID NO: 5, and the amino acid sequence of which is set forth in SEQ ID NO: 6). Furthermore, there is a possibility that there are spontaneous mutants of the Corl1 gene, such as allelic variants. Such mutants can also be used in the context of the present invention as indicators for identifying types of spinal neurons.

Thus, the "Corl1 gene" useful in the context of the present invention can be defined as an endogenous DNA selected from (1) to (4) as shown below.
(1) a DNA encoding a protein having the amino acid sequence of any one of SEQ ID NOs: 2, 4, or 6;
(2) a DNA having the nucleotide sequence of any one of SEQ ID NOs: 1, 3, or 5;
(3) a DNA encoding a protein having an amino acid sequence that includes a substitution, deletion, insertion and/or addition of one or more amino acids in the amino acid sequence of any one of SEQ ID NOs: 2, 4, or 6; and
(4) a vertebrate counterpart DNA of a DNA having the nucleotide sequence of any one of SEQ ID NOs: 1, 3, or 5.

When compared to the nucleotide sequence of the Corl1 gene of any one of SEQ ID NOs: 1, 3, or 5, the number of mutations in spontaneous mutants, such as allelic variants, is typically within 10 amino acids (for example, within 5 amino acids, or within 3 amino acids) at an amino acid level.

Meanwhile, DNAs of other vertebrates, which are counterparts to a DNA of a particular vertebrate, in general have high homology to the DNA of the particular vertebrate. The phrase "high homology" means a sequence homology of 50% or higher, preferably 70% or higher, more preferably 80% or higher, even more preferably 90% or higher (for example, 95% or higher, or 96%, 97%, 98%, or 99% or higher). Such homology can be determined using mBLAST algorithm (Altschul et al. (1990) Proc. Natl. Acad. Sci. USA 87:2264-8; Karlin and Altschul (1993) Proc. Natl. Acad. Sci. USA 90:5873-7). Meanwhile, when isolated from the living body, such DNAs of other vertebrates, which are counterparts to a DNA of a particular vertebrate, would hybridize to the DNA of the particular vertebrate under stringent conditions. The stringent conditions include, for example, "2x SSC/0.1% SDS at 50°C", "2x SSC/0.1% SDS at 42°C", and "1x SSC/0.1% SDS at 37°C", and for more stringent conditions include "2x SSC/0.1% SDS at 65°C", "0.5x SSC/0.1% SDS at 42°C", and "0.2x SSC/0.1% SDS at 65°C".

The one or more polynucleotides that constitute the active component of a reagent of the present invention will hybridize to a transcript of an endogenous Corl1 gene.

Exemplary hybridization conditions include "2x SSC/0.1 % SDS at 50°C", "2x SSC/0.1% SDS at 42°C", and "1x SSC/0.1% SDS at 37°C", and for more stringent conditions include "2x SSC/0.1% SDS at 65°C", "0.5x SSC/0.1% SDS at 42°C", and "0.2x SSC, 0.1% SDS, 65°C". More specifically, the following method using Rapid-hyb buffer (Amersham Life Science) may be used: after 30 minutes or more of prehybridization at 68°C, a probe is added, and the membrane is incubated at 68°C for an hour or more to allow hybrid formation; then, the membrane is washed three times with 2x SSC/0.1% SDS at room temperature for 20 minutes, and then washed three times with 1x SSC/0.1% SDS at 37°C for 20 minutes; finally, the membrane is washed twice with 1x SSC/0.1% SDS at 50°C for 20 minutes. Alternatively, for example, the following procedure may be used: after 30 minutes or more of prehybridization using Expresshyb Hybridization Solution (CLONTECH) at 55°C, a labeled probe is added, and the membrane is incubated at 37 to 55°C for an hour or more; the membrane is washed three times with 2x SSC/0.1% SDS at room temperature for 20 minutes, and then once with 1x SSC/0.1% SDS at 37°C for 20 minutes. More stringent conditions can be achieved, for example, by increasing the temperature of prehybridization, hybridization, and/or the second washing. For example, the temperature of prehybridization and hybridization may be 60°C. The temperature may be 68°C to achieve furthermore stringent conditions. Those skilled in the art can determine the appropriate conditions by altering probe concentration and length, the nucleotide sequence constituent of the probe, reaction time, and the like in addition to the conditions of salt concentration of such buffers, temperature, and such.

In a preferred embodiment, the present invention relates to reagents for identifying types of spinal neurons, particularly those including as active component one or more polynucleotides that hybridize under stringent conditions to a polynucleotide having a nucleotide sequence selected from the group consisting of SEQ ID NOs: 1, 3, and 5.

The length of the one or more polynucleotides contained within a reagent of the present invention is not particularly limited so long as specific detection of the expression of the Corl1 gene is provided. In general, such polynucleotides have a nucleotide sequence composed of at least consecutive 15 nucleotides complementary to the nucleotide sequence of the Corl1 gene. Such polynucleotides may be used as probes for detecting the expression of Corl1 mRNA or as amplification primers for detecting Corl1 mRNA. When used as a probe, such a polynucleotide is composed of 15 to 100 nucleotides, and preferably 15 to 35 nucleotides. Alternatively, when used as a primer, such a polynucleotide is composed of at least 15 or more nucleotides, preferably about 30 nucleotides.

When used as a probe, the polynucleotide is labeled, if necessary, with a radioisotope, non-radioactive compound, or the like. Alternatively, when used as a primer, the polynucleotide may be designed to be complementary to its target sequence at its 3' end and to have a restriction enzyme recognition site, tag sequence, or such at its 5' end. Such polynucleotides, having a nucleotide sequence of at least consecutive 15 nucleotides, can hybridize to Corl1 mRNA.

If necessary, the polynucleotide may include non-naturally occurring nucleotides, for example, 4-acetyl cytidine, 5-(carboxyhydroxymethyl)uridine, 2'-O-methylcytidine, 5-carboxymethylaminomethyl-2-thiouridine, 5-carboxymethylaminomethyl uridine, dihydrouridine, 2'-O-methyl pseudouridine, β-D-galactosyl queuosine, 2'-O-methylguanosine, inosine, N6-isopentenyl adenosine, 1-methyladenosine, 1-methyl pseudouridine, 1-methylguanosine, 1-methyl inosine, 2,2-dimethylguanosine, 2-methyladenosine, 2-methylguanosine, 3-methylcytidine, 5-methylcytidine, N6-methyladenosine, 7-methylguanosine, 5-methylaminomethyl-uridine, 5-methoxyaminomethyl-2-thiouridine, β-D-mannosylqueuosine, 5-methoxycarbonylmethyl-2-thiouridine, 5-methoxycarbonylmethyl uridine, 5-methoxyuridine, 2-methylthio-N6-isopentenyl adenosine, N-((9-β-D-ribofuranosyl-2-methylriopurine-6-yl)carbamoyl)threonine, N-((9-β-D-ribofuranosylpurine-6-yl) N-methylcarbamoyl) threonine, uridine-5-oxyacetate methyl ester, uridine-5-oxyacetate, wybutoxosine, pseudouridine, queuosine, 2-thiocytidine, 5-methyl-2-thiouridine, 2-thiouridine, 4-thiouridine, 5-methyluridine, N-((9-p-D-ribofuranosylpurine-6-yl)carbamoyl) threonine, 2'-O-methyl-5-methyluridine, 2'-O-methyluridine, wybutosine, and 3-(3-amino-3-carboxypropyl)uridine.

The one or more polynucleotides that constitute the active component of a reagent of the present invention can also be produced by chemical synthesis based on the known sequence of Corl1. Alternatively, such polynucleotides can be prepared from Corl1 gene-expressing cells using hybridization, PCR, or such.

In accordance with the present invention, the Corl1 gene may be used in combination with other known markers to identify types of spinal neurons. This allows for more precise identification of various types of spinal neurons. Thus, the present invention also provides kits for identifying types of spinal neurons, such kits including, in combination, one or more of the above-described polynucleotides that hybridize to a transcript of the Corl1 gene and the polynucleotides that hybridize to transcripts of one or more genes selected from the group consisting of Brn3a, Pax2, Lbx1, Lim1, Lim2, LH2A, LH2B, Isl1, Lmx1b, Tlx1 (Nat Rev Neurosci. 2003 Apr; 4(4):289-97 Caspary T, Anderson KV. Patterning cell types in the dorsal spinal cord: what the mouse mutants say.), and Tlx3 (Nat Rev Neurosci. 2003 Apr; 4(4):289-97 Caspary T, Anderson KV.).

In a preferred embodiment, the present invention provides kits for identifying types of spinal neurons, such kits including as active component one or more polynucleotides that hybridize under stringent conditions to polynucleotides having nucleotide sequence selected from the group consisting of SEQ ID NOs: 1, 3, and 5, and polynucleotides that hybridize under stringent conditions to the transcripts of one or more genes selected from the group consisting of Brn3a, Pax2, Lbx1, Lim1, Lim2, LH2A, LH2B, Isl1, Lmx1b, Tlx1, and Tlx3.

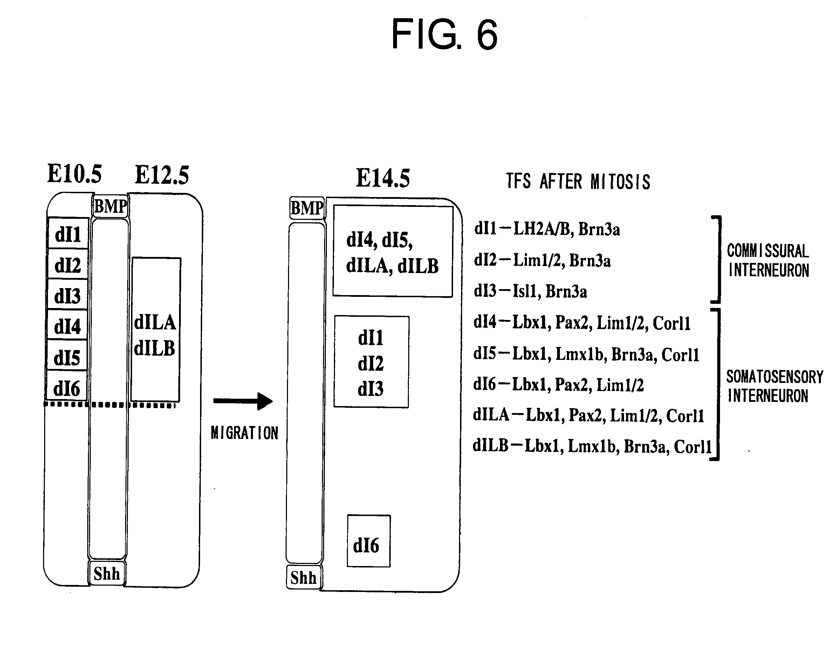

Many marker gene sequences are known in the art, as shown below. The specificity of expression of such marker genes in spinal neurons is shown in Fig. 6.

The nucleotide sequence of mouse Brn3a is set forth in SEQ ID NO: 7, and the amino acid sequence is set forth in SEQ ID NO: 8. The nucleotide sequence of human Brn3a is set forth in SEQ ID NO: 9, and the amino acid sequence is set forth in SEQ ID NO: 10. The nucleotide sequence of rat Brn3a is set forth in SEQ ID NO: 11, and the amino acid sequence is set forth in SEQ ID NO: 12. Herein, like the Corl1 gene, the Brn3a gene is defined as an endogenous DNA selected from (1) to (4) as shown below.
(1) a DNA encoding a protein having the amino an acid sequence of any one of SEQ ID NOs: 8, 10, or 12;
(2) a DNA having the nucleotide sequence of any one of SEQ ID NOs: 7, 9, or 11;
(3) a DNA encoding a protein having an amino acid sequence that includes a substitution, deletion, insertion, and/or addition of one or more amino acids in the amino acid sequence of any one of SEQ ID NOs: 8, 10, or 12; and
(4) a vertebrate counterpart DNA of a DNA having the nucleotide sequence of any one of SEQ ID NOs: 7, 9, or 11.

The nucleotide sequence of mouse Pax2 is set forth in SEQ ID NO: 13, and the amino acid sequence is set forth in SEQ ID NO: 14. The nucleotide sequence of human Pax2 is set forth in SEQ ID NO: 15, and the amino acid sequence is set forth in SEQ ID NO: 16.
In the present invention, Pax2 gene is defined as an endogenous DNA selected from (1) to (4) as shown below.
(1) a DNA encoding a protein having the amino acid sequence of SEQ ID NO: 14 or 16;
(2) a DNA having the nucleotide sequence of SEQ ID NO: 13 or 15;
(3) a DNA encoding a protein having an amino acid sequence that includes a substitution, deletion, insertion, and/or addition of one or more amino acids in the amino acid sequence of SEQ ID NO: 14 or 16; and
(4) a vertebrate counterpart DNA of a DNA having the nucleotide sequence of SEQ ID NO: 13 or 15.

The nucleotide sequence of mouse Lbx1 is set forth in SEQ ID NO: 17, and the amino acid sequence is set forth in SEQ ID NO: 18. The nucleotide sequence of human Lbx1 is set forth in SEQ ID NO: 19, and the amino acid sequence is set forth in SEQ ID NO: 20.
In the context of the present invention, Lbx1 gene is defined as an endogenous DNA selected from (1) to (4) as shown below.
(1) a DNA encoding a protein having the amino acid sequence of SEQ ID NO: 18 or 20;
(2) a DNA having the nucleotide sequence of SEQ ID NO: 17 or 19;
(3) a DNA encoding a protein having an amino acid sequence that includes a substitution, deletion, insertion, and/or addition of one or more amino acids in the amino acid sequence of SEQ ID NO: 18 or 20; and
(4) a vertebrate counterpart DNA of a DNA having the nucleotide sequence of SEQ ID NO: 17 or 19.

The nucleotide sequence of mouse Lim1 is set forth in SEQ ID NO: 21, and the amino acid sequence is set forth in SEQ ID NO: 22. The nucleotide sequence of human Lim1 is set forth in SEQ ID NO: 23, and the amino acid sequence is set forth in SEQ ID NO: 24.
In the context of the present invention, Lim1 gene is defined as an endogenous DNA selected from (1) to (4) as shown below.
(1) a DNA encoding a protein having the amino acid sequence of SEQ ID NO: 22 or 24;
(2) a DNA having the nucleotide sequence of SEQ ID NO: 21 or 23;
(3) a DNA encoding a protein having an amino acid sequence that includes a substitution, deletion, insertion, and/or addition of one or more amino acids in the amino acid sequence of SEQ ID NO: 22 or 24; and
(4) a vertebrate counterpart DNA of a DNA having the nucleotide sequence of SEQ ID NO: 21 1 or 23.

The nucleotide sequence of mouse Lim2 is set forth in SEQ ID NO: 25, and the amino acid sequence is set forth in SEQ ID NO: 26. The nucleotide sequence of human Lim2 is set forth in SEQ ID NO: 27, and the amino acid sequence is set forth in SEQ ID NO: 28. The nucleotide sequence of rat Lim2 is set forth in SEQ ID NO: 29, and the amino acid sequence is set forth in SEQ ID NO: 30. In the context of the present invention, Lim2 gene is defined as an endogenous DNA selected from (1) to (4) as shown below.
(1) a DNA encoding a protein having the amino acid sequence of any one of SEQ ID NOs: 26, 28, or 30;
(2) a DNA having the nucleotide sequence of any one of SEQ ID NOs: 25, 27, or 29;
(3) a DNA encoding a protein having an amino acid sequence that includes a substitution, deletion, insertion, and/or addition of one or more amino acids in the amino acid sequence of any one of SEQ ID NOs: 26, 28, or 30; and
(4) a vertebrate counterpart DNA of a DNA having the nucleotide sequence of any one of SEQ ID NOs: 25, 27, or 29.

The nucleotide sequence of mouse Isl1 is set forth in SEQ ID NO: 31, and the amino acid sequence is set forth in SEQ ID NO: 32. The nucleotide sequence of human Isl1 is set forth in SEQ ID NO: 33, and the amino acid sequence is set forth in SEQ ID NO: 34. The nucleotide sequence of rat Isl1 is set forth in SEQ ID NO: 35, and the amino acid sequence is set forth in SEQ ID NO: 36. In the context of the present invention, Isl1 gene is defined as an endogenous DNA selected from (1) to (4) as shown below.
(1) a DNA encoding a protein having the amino acid sequence of any one of SEQ ID NOs: 32, 34, or 36;
(2) a DNA having the nucleotide sequence of any one of SEQ ID NOs: 31, 33, or 35;
(3) a DNA encoding a protein having an amino acid sequence that includes a substitution, deletion, insertion, and/or addition of one or more amino acids in the amino acid sequence of any one of SEQ ID NOs: 32, 34, or 36; and
(4) a vertebrate counterpart DNA of a DNA having the nucleotide sequence of any one of SEQ ID NOs: 31, 33, or 35.

The nucleotide sequence of mouse LH2A is set forth in SEQ ID NO: 37, and the amino acid sequence is set forth in SEQ ID NO: 38. The nucleotide sequence of human LH2A is set forth in SEQ ID NO: 39, and the amino acid sequence is set forth in SEQ ID NO: 40.

In the context of the present invention, LH2A gene is defined as an endogenous DNA selected from (1) to (4) as shown below.
(1) a DNA encoding a protein having the amino acid sequence of SEQ ID NO: 38 or 40;
(2) a DNA having the nucleotide sequence of SEQ ID NO: 37 or 39;
(3) a DNA encoding a protein having an amino acid sequence that includes a substitution, deletion, insertion, and/or addition of one or more amino acids in the amino acid sequence of SEQ ID NO: 38 or 40; and
(4) a vertebrate counterpart DNA of a DNA having the nucleotide sequence of SEQ ID NO: 37 or 39.

The nucleotide sequence of mouse LH2B is set forth in SEQ ID NO: 41, and the amino acid sequence is set forth in SEQ ID NO: 42. The nucleotide sequence of human LH2B is set forth in SEQ ID NO: 43, and the amino acid sequence is set forth in SEQ ID NO: 44. The nucleotide sequence of rat LH2B is set forth in SEQ ID NO: 45, and the amino acid sequence is set forth in SEQ ID NO: 46. In the context of the present invention, LH2B gene is defined as an endogenous DNA selected from (1) to (4) as shown below.
(1) a DNA encoding a protein having the amino acid sequence of any one of SEQ ID NOs: 42, 44, or 46;
(2) a DNA having the nucleotide sequence of any one of SEQ ID NOs: 41, 43, or 45;
(3) a DNA encoding a protein having an amino acid sequence that includes a substitution, deletion, insertion, and/or addition of one or more amino acids in the amino acid sequence of any one of SEQ ID NOs: 42, 44, or 46; and
(4) a vertebrate counterpart DNA of s DNA having the nucleotide sequence of any one of SEQ ID NOs: 41, 43, or 45.

The nucleotide sequence of mouse Lmx1b is set forth in SEQ ID NO: 47, and the amino acid sequence is set forth in SEQ ID NO: 48. The nucleotide sequence of human Lmx1b is set forth in SEQ ID NO: 49, and the amino acid sequence is set forth in SEQ ID NO: 50. The nucleotide sequence of rat Lmx1b is set forth in SEQ ID NO: 51, and the amino acid sequence is set forth in SEQ ID NO: 52. In the context of the present invention, Lmx1b gene is defined as an endogenous DNA selected from (1) to (4) as shown below.
(1) a DNA encoding a protein having the amino acid sequence of any one of SEQ ID NOs: 48, 50, or 52;
(2) a DNA having the nucleotide sequence of any one of SEQ ID NOs: 47, 49, or 51;
(3) a DNA encoding a protein having an amino acid sequence that includes a substitution, deletion, insertion, and/or addition of one or more amino acids in the amino acid sequence of any one of SEQ ID NOs: 48, 50, or 52; and
(4) a vertebrate counterpart DNA of a DNA having the nucleotide sequence of any one of SEQ ID NOs: 47, 49, or 51.

The nucleotide sequence of mouse Tlx1 is set forth in SEQ ID NO: 53, and the amino acid sequence is set forth in SEQ ID NO: 54. The nucleotide sequence of human Tlx1 is set forth in SEQ ID NO: 55, and the amino acid sequence is set forth in SEQ ID NO: 56.

In the context of the present invention, Tlx1 gene is defined as an endogenous DNA selected from (1) to (4) as shown below.
(1) a DNA encoding a protein having the amino acid sequence of SEQ ID NO: 54 or 56;
(2) a DNA having the nucleotide sequence of SEQ ID NO: 53 or 55;
(3) a DNA encoding a protein having an amino acid sequence that includes a substitution, deletion, insertion, and/or addition of one or more amino acids in the amino acid sequence of SEQ ID NO: 54 or 56; and
(4) a vertebrate counterpart DNA of a DNA having the nucleotide sequence of SEQ ID NO: 53 or 55.

The nucleotide sequence of mouse Tlx3 is set forth in SEQ ID NO: 57, and the amino acid sequence is set forth in SEQ ID NO: 58. The nucleotide sequence of human Tlx3 is set forth in SEQ ID NO: 59, and the amino acid sequence is set forth in SEQ ID NO: 60.
In the context of the present invention, Tlx3 gene is defined as an endogenous DNA selected from (1) to (4) shown below.
(1) a DNA encoding a protein having the amino acid sequence of SEQ ID NO: 58 or 60;
(2) a DNA having the nucleotide sequence of SEQ ID NO: 57 or 59;
(3) a DNA encoding a protein having an amino acid sequence that includes a substitution, deletion, insertion, and/or addition of one or more amino acids in the amino acid sequence of SEQ ID NO: 58 or 60; and
(4) a vertebrate counterpart DNA of s DNA having the nucleotide sequence of SEQ ID NO: 57 or 59.

The kits of the present invention may include, in addition to the above-described polynucleotides, one or more reagents for detecting the expression of the transcripts of Corl1 and other marker genes, buffers, and such, as necessary. In addition, instructions and other descriptions for use of the kits may be included in the package.

The spinal neuron-specific expression of the Corl1 gene was demonstrated not only at the transcriptional level, as described above, but also at the translational level. Thus, the present invention also provides reagents for identifying types of spinal neurons, such reagents including as active component an antibody that binds to the translation product of the Corl1 gene. Furthermore, in a preferred embodiment, the present invention relates to reagents for identifying types of spinal neurons, such reagents including as active component an antibody that binds to a polypeptide having an amino acid sequence selected from the group consisting of SEQ ID NOs: 2, 4, and 6, or a partial sequence thereof.

The antibodies that constitute the active component of a reagent of the present invention include polyclonal antibodies, monoclonal antibodies, chimeric antibodies, single-chain antibodies (scFv; Huston et al. (1988) Proc. Natl. Acad. Sci. USA 85: 5879-83; The Pharmacology of Monoclonal Antibody, Vol. 113, Rosenburg and Moore ed., Springer Verlag (1994) pp. 269-315), humanized antibodies, multispecific antibodies (LeDoussal et al. (1992) Int. J. Cancer Suppl. 7: 58-62; Paulus (1985) Behring Inst. Mitt. 78: 118-32; Millstein and Cuello (1983) Nature 305: 537-9; Zimmermann (1986) Rev. Physiol. Biochem. Pharmacol. 105: 176-260; VanDijk et al. (1989) Int. J. Cancer 43: 944-9), and antibody fragments, such as Fab, Fab', F(ab')₂, Fc, and Fv and the like. Such antibodies may be modified by PEG or such, if required. The antibodies may be produced as fusion proteins with β-galactosidase, maltose-binding protein, GST, green fluorescence protein (GFP), or such so that the detection can be achieved without using any secondary antibodies. Alternatively, the antibodies may be altered by labeling them with biotin, or such so that the antibodies can be detected and recovered by using avidin, streptavidin, or the like.

Polyclonal antibodies can be obtained from, for example, the serum collected from immunized animals, more particularly mammals immunized with purified Corl1 polypeptides or fragments thereof, coupled with adjuvants as necessary. Although there are no particular limitations as to the mammals used, typical examples include rodents, lagomorphs and primates. Specific examples include rodents such as mice, rats and hamsters, lagomorphs such as rabbits, and primates such as monkeys, including cynomolgus monkeys, rhesus monkeys, baboons and chimpanzees. Animals can be immunized by suitably diluting and suspending a sensitizing antigen in phosphate-buffered saline (PBS) or physiological saline, mixing with an adjuvant as necessary until emulsified, and injecting into an animal, either intraperitoneally or subcutaneously. In a preferred embodiment, the sensitizing antigens mixed with Freund's incomplete adjuvant are administered several times every four to 21 days. Antibody production can be confirmed using conventional methods to measure the level of an antibody of interest in the serum. Finally, the serum itself may be used as a polyclonal antibody, or it may be further purified. See, for example, "Current Protocols in Molecular Biology" (John Wiley & Sons (1987) Sections 11.12-11.13) for specific methods.

Monoclonal antibodies can be produced by removing the spleen of an animal immunized in a manner described above, separating immunocytes from the spleen, and fusing them with a suitable myeloma cell using polyethylene glycol (PEG) or such to establish hybridomas. Cell fusion can be carried out according to the Milstein method (Galfre and Milstein (1981) Methods Enzymol. 73: 3-46). Cells that allow chemical selection of fused cells are particularly preferred myeloma cells. When using such myeloma cells that allow chemical selection, fused hybridomas can be selected by culturing in a culture medium (HAT culture medium) that contains hypoxanthine, aminopterin, and thymidine, which destroys non-fused cells. Next, clones that produce antibodies against Corl1 polypeptides, or fragments thereof, are selected from the established hybridomas. The selected clones are then introduced into the abdominal cavities of mice or such, and ascites is collected to obtain the monoclonal antibodies. For information on specific methods see "Current Protocols in Molecular Biology" (John Wiley & Sons (1987) Section 11.4-11.11).

Hybridomas can also be obtained by first using an immunogen to sensitize human lymphocytes that have been infected *in vitro* with EB virus, then fusing the sensitized lymphocytes with human myeloma cells (such as U266) to obtain hybridomas that produce human antibodies (Japanese Patent Application Kokai Publication No. (JP-A) S63-17688 (unexamined, published Japanese patent application)). In addition, human antibodies can also be obtained by using antibody-producing cells generated by sensitizing transgenic animals which have the repertoire of human antibody genes (WO92/03918; WO93/02227; WO94/02602; WO94/25585;; WO96/34096; Mendez et al. (1997) Nat. Genet. 15: 146-156, etc.). Methods that do not use hybridomas can be exemplified by methods in which cancer genes are introduced to immortalize immunocytes, such as antibody-producing lymphocytes.

In addition, antibodies can also be produced using genetic recombination techniques (see Borrebaeck and Larrick (1990) Therapeutic Monoclonal Antibodies, MacMillan Publishers Ltd., UK). First, a gene that encodes an antibody is cloned from hybridomas or other antibody-producing cells (such as sensitized lymphocytes). The resulting gene is then inserted into a suitable vector, the vector is introduced into a host, and the host is cultured to produce the antibody. This type of recombinant antibody is also included in the active component of the reagent of the present invention. Typical examples of recombinant antibodies include chimeric antibodies, which are composed of a non-human antibody-derived variable region and a human antibody-derived constant region, and humanized antibodies, which are composed of a non-human-derived antibody complementarity determining region (CDR), a human antibody-derived framework region (FR), and a human antibody constant region (Jones et al. (1986) Nature 321: 522-5; Reichmann et al. (1988) Nature 332: 323-9; Presta (1992) Curr. Op. Struct. Biol. 2: 593-6; Methods Enzymol. 203: 99-121 (1991)).

Antibody fragments can be produced by treating the aforementioned polyclonal or monoclonal antibodies with enzymes such as papain or pepsin. Alternatively, an antibody fragment can be produced through genetic engineering techniques using a gene that encodes an antibody fragment (see Co et al., (1994) J. Immunol. 152: 2968-76; Better and Horwitz (1989) Methods Enzymol. 178: 476-96; Pluckthun and Skerra (1989) Methods Enzymol. 178: 497-515; Lamoyi (1986) Methods Enzymol. 121: 652-63; Rousseaux et al. (1986) 121: 663-9; Bird and Walker (1991) Trends Biotechnol. 9: 132-7).

Multispecific antibodies include bispecific antibodies (BsAb), diabodies (Db), and such. Multispecific antibodies can be produced by methods such as (1) chemically coupling antibodies having different specificities with different types of bifunctional linkers (Paulus (1985) Behring Inst. Mitt. 78: 118-32), (2) fusing hybridomas that secrete different monoclonal antibodies (Millstein and Cuello (1983) Nature 305: 537-9), or (3) transfecting eukaryotic cell expression systems, such as mouse myeloma cells, with a light chain gene and a heavy chain gene of different monoclonal antibodies (four types of DNA), followed by the isolation of a bispecific monovalent portion (Zimmermann (1986) Rev. Physio. Biochem. Pharmacol. 105: 176-260; Van Dijk et al. (1989) Int. J. Cancer 43: 944-9). On the other hand, diabodies are dimer antibody fragments comprising two bivalent polypeptide chains that are constructed by gene fusion. These can be produced using known methods (see Holliger et al. (1993) Proc. Natl. Acad. Sci. USA 90: 6444-8; EP404097; WO93/11161).

Antibodies and antibody fragments can be recovered and purified using Protein A and Protein G. They can also be purified by the protein purification techniques described above, in the same way as for non-antibody polypeptides (Antibodies: A Laboratory Manual, Ed Harlow and David Lane, Cold Spring Harbor Laboratory (1988)). For example, when using Protein A to purify an antibody of the present invention, known Protein A columns such as Hyper D, POROS, or Sepharose F.F. (Pharmacia) can be used. The concentration of the resulting antibody can be determined by measuring absorbance or using an enzyme linked immunoadsorbent assay (ELISA).

The antigen binding activity of an antibody can be determined by measuring absorbance, or using fluorescent antibody methods, enzyme immunoassay (EIA) methods, radioimmunoassay (RIA) methods, or ELISA. When ELISA is used, a Corl1 polypeptide or fragment thereof is first immobilized onto a support, such as a plate. Then, a sample containing the antibody of interest is added. Herein, the samples containing an antibody of interest include, for example, culture supernatants of antibody-producing cells, purified antibodies, and such. Next, a secondary antibody that recognizes an antibody that is an active component of a reagent of the present invention is added, and the plate is incubated. The plate is then washed and the label attached to the secondary antibody is detected. Specifically, if a secondary antibody is labeled with alkaline phosphatase, for example, its antigen binding activity can be determined by adding an enzyme substrate such as p-nitrophenyl phosphate, and then measuring the absorbance. In addition, a commercially available system such as BIAcore (Pharmacia) can also be used to evaluate antibody activities.

The translation products of other known marker genes may be used as targets in combination with the translation product of the Corl1 gene to identify types of spinal neurons in accordance the present invention. Thus, the present invention also provides kits for identifying types of spinal neurons, such kits including, in combination, one or more antibodies that bind to the translation product of the Corl1 gene and one or more antibodies that bind to the translation product of one or more genes selected from the group consisting of Brn3a, Pax2, Lbx1, Lim1, Lim2, LH2A, LH2B, Isl1, Lmx1b, Tlx1, and Tlx3.

In a further preferred embodiment, the present invention relates to kits for identifying types of spinal neurons, such kits including one or more antibodies that bind to a polypeptide having an amino acid sequence selected from the group consisting of SEQ ID NOs: 2, 4, and 6, or a partial sequence thereof, and one or more antibodies that bind to the translation product of a gene selected from the group consisting of Brn3a, Pax2, Lbx1, Lim1, Lim2, LH2A, LH2B, Isl1, Lmx1b, Tlx1, and Tlx3.

The kits of the present invention may include, in addition to the above-described antibodies, reagents for detecting binding activity, buffers, and the like, if necessary. In addition, instructions and other descriptions for use of the kits may be included in the package.

The present invention also provides methods for identifying types of spinal neurons, comprising the steps of detecting a transcript or translation product of the Corl1 gene in spinal neurons.

The detection of a transcript of the Corl1 gene by the method of the present invention can be made by contacting the polynucleotide of the present invention described above with nucleic acid extract of cell samples that would contain spinal cord interneurons and detecting nucleic acid which hybridizes to the polynucleotide in the nucleic acid extract,.

The polynucleotide probe is preferably labeled with radioisotope or non-radioactive compound to detect a transcript of the Corl1 gene. Such radioisotopes to be used as a label include for example, ³⁵S, and ³H. When a radiolabeled polynucleotide probe is used, RNA that binds to a marker can be detected by detecting silver particles by emulsion autoradiography. Meanwhile, as for conventional non-radioisotopic compounds that are used to label polynucleotide probes include biotin and digoxigenin are known. The detection of biotin-labeled markers can be achieved, for example, using fluorescent labeled avidin or avidin labeled with an enzyme, such as alkaline phosphatase or horseradish peroxidase. On the other hand, the detection of digoxigenin-labeled markers can be achieved by using fluorescent labeled anti-digoxigenin antibody or anti-digoxigenin antibody labeled with an enzyme, such as alkaline phosphatase or horseradish peroxidase. When enzyme labeling is used, the detection can be made by allowing stable dye to deposit at marker positions by incubating with an enzyme substrate.

When polynucleotide primers are used for detection of a transcript of the Corl1 gene, Corl1 gene transcripts can be detected by amplifying nucleic acid that hybridizes to the polynucleotide primers, for example, using techniques such as RT-PCR.

The detection of translation products of the Corl1 gene with the methods of the present invention can be made by contacting the antibody described above with protein extract of cell samples that would contain spinal cord interneurons and then detecting proteins bound to the antibody. As described above, assay methods for antigen binding activities of antibodies include absorbance measurement, fluorescent antibody method, enzyme immunoassay (EIA), radioimmunoassay (RIA), and ELISA and the like.

In the context of the present invention, detailed spinal neuron types can be identified by detecting, in addition to a transcript or translation product of the Corl1 gene, the transcripts or translation products of one or more genes selected from the group consisting of Brn3a, Pax2, Lbx1, Lim1, Lim2, LH2A, LH2B, Isl1, Lmx1b, Tlx1, and Tlx3. Such methods are also included in the present invention.

In a preferred embodiment, methods of the present invention for identifying types of spinal neurons include the steps of contacting spinal neurons with:
(1) a polynucleotide that hybridizes under stringent conditions to a polynucleotide having a nucleotide sequence selected from the group consisting of SEQ ID NOs: 1, 3, and 5; or
(2) an antibody that binds to a polypeptide having an amino acid sequence selected from the group consisting of SEQ ID NOs: 2, 4, and 6, or a partial sequence thereof.

In a more preferred embodiment, in addition to the steps described above, the method further includes the steps of contacting spinal neurons with:
(1) a polynucleotide that hybridizes under stringent conditions to a transcript of at least one gene selected from the group consisting of Brn3a, Pax2, Lbx1, Lim1, Lim2, LH2A, LH2B, Isl1, Lmx1b, Tlx1, and Tlx3; or
(2) an antibody that binds to a translation product of at least one gene selected from the group consisting of Brn3a, Pax2, Lbx1, Lim1, Lim2, LH2A, LH2B, Isl1, Lmx1b, Tlx1, and Tlx3.

In a further a preferred embodiment, in addition to the above-described steps, the present invention also provides methods including the step of discriminating the group consisting of at least one spinal neuron type selected from dI1, dI2, dI3, and dI6 and the group consisting of at least one spinal neuron type selected from dI4, dI5, dILA, and dILB.

In a further a preferred embodiment, the present invention also provides methods for identifying types of spinal neurons, such methods including the step of discriminating between spinal neuron types that do express a transcript of one or more genes selected from the group consisting of Brn3a, Pax2, Lbx1, Lim1, Lim2, LH2A, LH2B, Isl1, Lmx1b, Tlx1, and Tlx3 and spinal cord cell types that do not express a transcript of one or more genes selected from the group consisting of Brn3a, Pax2, Lbx1, Lim1, Lim2, LH2A, LH2B, Isl1, Lmx1b, Tlx1, and Tlx3.

Since Corl1 is specifically expressed in differentiated spinal cord interneurons, it can be used in the screening for reagents that induce the differentiation of spinal neurons. Specifically, whether a test sample has the ability to induce differentiation of cells that have a potential to differentiate into spinal neurons can be determined by inducing the differentiation of cells that have a potential to differentiate into spinal neurons in the presence of the test sample and then detecting the expression of Corl1 in the differentiated cells. Thus, the present invention provides methods for screening candidate compounds for reagents that induce differentiation into spinal neurons, such methods using the expression of Corl1 as an indicator and including the steps of:
(a) inducing cells that have a potential to differentiate into spinal neurons to differentiate into spinal neurons in the presence of a test sample;
(b) detecting a transcript or translation product of the Corl1 gene in the differentiation induced cells; and
(c) selecting those test samples that increase the level of the transcript or translation product when compared with the level detected in the absence of test samples.

"Cells that have a potential to differentiate into spinal neurons" are preferably cells samples that contain cells such as ES cells having pluripotency, which can be differentiated into spinal neurons. Methods for inducing differentiation into spinal neurons *in vitro,* in which known ES cells, bone marrow stromal cells, immortalized cells derived from neuron, are known in the art (Japanese Patent Kohyo Publication No. (JP-A) H8-509215 (unexamined Japanese national phase publication corresponding to a non-Japanese international publication); Japanese Patent Kohyo Publication No. (JP-A) H11-506930 (unexamined Japanese national phase publication corresponding to a non-Japanese international publication); Japanese Patent Kohyo Publication No. (JP-A) 2002-522070 (unexamined Japanese national phase publication corresponding to a non-Japanese international publication)), and neural stem cells (Japanese Patent Kohyo Publication No. (JP-A) H11-509729 (unexamined Japanese national phase publication corresponding to a non-Japanese international publication)) are used as starter material cells.

The test sample to be contacted with the cells may be any compound which includes, for example, gene libraries of expression products, libraries of synthetic low-molecular-weight compounds, synthetic peptide libraries, antibodies, substances released from bacteria, cell extract (microorganisms, plant cells, and animal cells), cell culture supernatants (microorganisms, plant cells, and animal cells), purified or partially purified polypeptides, marine organisms, extract derived from plants and animals and the like, soil, and random phase peptide display libraries.

As described above, a transcript or translation product of the Corl1 gene can be detected using polynucleotides that hybridize to Corl transcripts or antibodies that bind to Corl translation products.

The differentiation of cells can be assessed by comparing the expression level of Corl1 in the absence of the test sample. Specifically, when a test sample increases the level of a transcript or translation product of the Corl1 gene as compared with the level determined in the absence of the test sample, it can be determined that the test sample has the ability to induce differentiation into spinal neurons. Herein, "increase" means, for example, a two-fold increase, preferably five-fold increase, more preferably an increase of 10-fold or more.

The test samples selected through screening, using the methods of the present invention, find utility as reagents for inducing differentiation into spinal neurons and thus are candidates for therapeutic drugs for diseases associated with a deficiency in spinal neurons.

Furthermore, the present invention relates to the uses of (a) or (b) as described below in the production of reagents for identifying types of spinal neurons:
(a) a polynucleotide that hybridizes to a transcript of Corl1 gene; and
(b) an antibody that binds to a translation product of Corl1 gene.

All prior-art documents cited herein have been incorporated herein by reference.

### Examples

Hereinbelow, the present invention is specifically described with reference to Examples; however, it should not be construed as being limited thereto.

### [Example 1] Isolation and sequencing of Corl1

Genes whose expression levels were different between the ventral and dorsal regions of E12.5 mouse midbrain were identified by the subtraction (N-RDA) method to isolate embryonic brain region-specific genes. One of isolated fragments was a cDNA fragment encoding a protein whose function was unknown.

### 1 N-RDA method

### 1-1. Adapter preparation

The following oligonucleotides were annealed to each other, and prepared at 100 µM: (ad2: ad2S+ad2A, ad3: ad3S+ad3A, ad4: ad4S+ad4A, ad5: ad5S+ad5A, ad13: ad13S+ad13A)
ad2S: cagctccacaacctacatcattccgt (SEQ ID NO: 61)
ad2A: acggaatgatgt (SEQ ID NO: 62)
ad3S: gtccatcttctctctgagactctggt (SEQ ID NO: 63)
ad3A: accagagtctca (SEQ ID NO: 64)
ad4S: ctgatgggtgtcttctgtgagtgtgt (SEQ ID NO: 65)
ad4A: acacactcacag (SEQ ID NO: 66)
ad5S: ccagcatcgagaatcagtgtgacagt (SEQ ID NO: 67)
ad5A: actgtcacactg (SEQ ID NO: 68)
ad13S: gtcgatgaacttcgactgtcgatcgt (SEQ ID NO: 69)
ad13A: acgatcgacagt (SEQ ID NO: 70).

### 1-2. cDNA synthesis

Ventral and dorsal midbrain regions were cut out of E12.5 mouse embryos (Japan SLC). Total RNA was prepared using an RNeasy Mini Kit (Qiagen), and double-stranded cDNA was synthesized using a cDNA Synthesis Kit (Takara). After digestion with restriction enzyme RsaI, ad2 was added. ad2S was used as the primer to amplify the cDNA using 15 PCR cycles. The conditions for amplification were: a 5-minute incubation at 72°C; 15 reaction cycles of 30 seconds at 94°C, 30 seconds at 65°C and two minutes at 72°C; and finally a two-minute incubation at 72°C. In all cases, N-RDA PCR was carried out using a reaction solution containing the following components.

| | |
|---|---|
| 10x ExTaq | 5 µl |
| 2.5 mM dNTP | 4 µl |
| ExTaq | 0.25 µl |
| 100 µM primer | 0.5 µl |
| cDNA | 2 µl |
| Distilled water | 38.25 µl |

### 1-3. Driver production

The ad2S-amplified cDNA was further amplified by five PCR cycles. The conditions for amplification were: incubation at 94°C for two minutes; five reaction cycles of 30 seconds at 94°C, 30 seconds at 65°C and two minutes at 72°C; and a final two-minute incubation at 72°C. The cDNA was purified using a Qiaquick PCR Purification Kit (Qiagen), and digested with RsaI. 3 µg was used for each round of subtraction.

### 1-4. Tester production

The ad2S amplified cDNA was further amplified by five PCR cycles. The conditions for amplification were: incubation at 94°C for two minutes; five reaction cycles of 30 seconds at 94°C, 30 seconds at 65°C and two minutes at 72°C; and a final two-minute incubation at 72°C. The cDNA was purified using a Qiaquick PCR Purification Kit (Qiagen), and digested with RsaI. ad3 was added to 60 ng of the RsaI-digested cDNA.

### 1-5. First round of subtraction

The tester and driver produced in Sections 1-3 and 1-4 above were mixed, ethanol precipitated, and then dissolved in 1 µl of 1x PCR buffer. After a five-minute incubation at 98°C, 1 µl of 1x PCR buffer + 1M NaCl was added. After another five-minute incubation at 98°C, the tester and driver were hybridized at 68°C for 16 hours.

With ad3S as the primer, the hybridized cDNA was amplified by ten cycles of DNA (incubation at 72°C for five minutes; then ten reaction cycles of 30 seconds at 94°C, 30 seconds at 65°C and two minutes at 72°C). Next, the amplified cDNA was digested with Mung Bean Nuclease (Takara) and purified using a Qiaquick PCR Purification Kit. Then, it was amplified by 13 PCR cycles. The conditions for amplification were: incubation at 94°C for two minutes; 13 reaction cycles of 30 seconds at 94°C, 30 seconds at 65°C and two minutes at 72°C; and a final two-minute incubation at 72°C.

### 1-6. Normalization

1 µl of 2x PCR buffer was added to 8 ng of the cDNA amplified in the first round of subtraction. After incubating at 98°C for five minutes, 2 µl of 1x PCR buffer + 1 M NaCl was added. After another five minutes of incubation at 98°C, the cDNA was hybridized at 68°C for 16 hours.

The hybridized cDNA was digested with RsaI and then purified using a Qiaquick PCR Purification Kit. This was then amplified by eleven PCR cycles using ad3S as the primer (incubation at 94°C for two minutes; then eleven reaction cycles of 30 seconds at 94°C, 30 seconds at 65°C and two minutes at 72°C; and a final two-minute incubation at 72°C). The PCR product was then digested with RsaI and ad4 was then added.

### 1-7. Second Round of Subtraction

20 ng of the cDNA to which ad4 was added in Section 1-6 above was used as the tester and mixed with the driver of 1-3 above. The same subtraction procedure as used in Section 1-5 above was performed. Finally, ad5 was added to the cDNA following RsaI digestion.

### 1-8. Third Round of Subtraction

2 ng of the cDNA to which ad5 was added in Section 1-7 above was used as the tester and mixed with the driver of 1-3 above. The same subtraction procedure as used in section 1-5 above was performed. Finally, ad13 was added to the cDNA following RsaI digestion.

### 1-9. Fourth Round of Subtraction

2 ng of the cDNA to which ad13 was added in Section 1-8 above was used as the tester and mixed with the driver of 1-3 above. The same subtraction procedure as used in Section 1-5 above was performed. The amplified cDNA was cloned into pCRII vector (Invitrogen) and its nucleotide sequence was analyzed using the ABI3100 sequence analyzer.

### 2. Determination of full length cDNA sequence

BLAST searches were carried out using the sequence of cDNA fragment obtained by N-RDA. As a result, it was revealed that this gene encodes a protein whose function was unknown (Genbank accession No.: NM-172446). Accordingly, primers were designed based on the deposited sequence. The full-length cDNA was then cloned by RT-PCR.

Brain tissues, including diencephalon, midbrain and afterbrain, were excised from day 12.5 mouse embryos. Total RNA was prepared using RNeasy Mini kit (Qiagen). Single-stranded cDNA was synthesized using RNA PCR kit (TAKARA). The cDNA was used as a template. The thermal cycling profile used was follows: 5 minutes of incubation at 94°C, 35 cycles of 94°C for 30 seconds, 65°C for 30 seconds, and 72°C for 5 minutes, followed by incubation at 72°C for 2 minutes. The composition of PCR mixture used was as follows:
10x buffer 5 µl
2.5 mM dNTP 4 µl
Pyrobest polymerase (TAKARA) 0.5 µl
100 µM primer 0.5 µl
cDNA 1 µl
DMSO 2.5 µl
distilled water 36 µl
primer sequence
Corl1 F1: GAGGTCGACATGGCATTGCTGTGTGGCCTTGGGAG (SEQ ID NO: 71)
Corl1 R1: GAGGTCGACCTAGGGCAGCAGCGGAGGCTTGAAGG (SEQ ID NO: 72)

The amplified cDNA was cloned into pCRII (Invitrogen) and nucleotide sequence was determined using ABI3100 sequencer. The cDNA was found to encode 936 amino acids. This gene was named as Corl1.

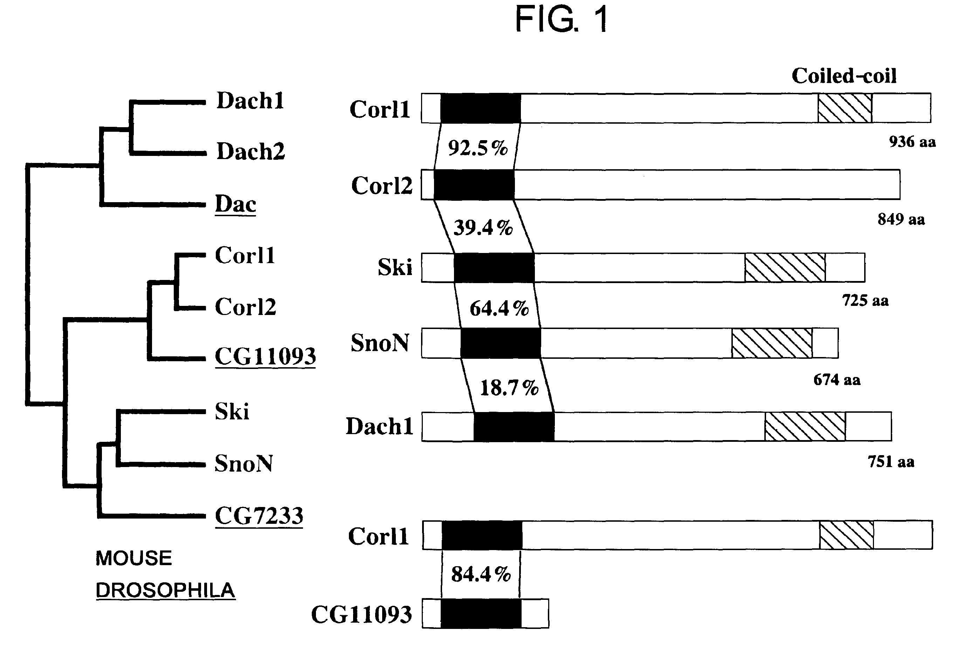

BLAST homology search was carried out using the amino acid sequence for Corl1. The results revealed that Corl1 was a protein exhibiting high homology to Ski, SnoN, and Dach (Fig. 1). In addition, a gene with an unknown function was also found to exhibit high homology to Corl1. This gene was named as Corl2. A Drosophila gene (CG11093) exhibiting high homology to Corl1 was also found, and thus it suggested that the gene has a function which is evolutionarily conserved.

### [Example 2] Analysis of Corll expression

In the next step, the expression of Corl1 was analyzed. First, the expression in tissues of adult mouse was analyzed by RT-PCR.

Single-stranded cDNA was synthesized from total RNA of each tissue (Promega) using RNA PCR kit (TAKARA), which was used as a template. The thermal cycling profile used was as follows:2 minutes of incubation at 94°C, 35 cycles of 94°C for 30 seconds, 65°C for 30 seconds, and 72°C for 30 seconds, followed by incubation at 72°C for 2 minutes. The composition of PCR mixture used was as follows:
10x buffer 1 µl
2.5 mM dNTP 0.8 µl
ExTaq 0.05 µl
100 µM primer 0.1 µl
cDNA 1 µl
distilled water 7.05 µl
primer sequence
Corl1 F2: ATGCAGAGAGCATCGCTAAGCTCTAC (SEQ ID NO: 73)
Corl1 R2: AAGCGGTTGGACTCTACGTCCACCTC (SEQ ID NO: 74)

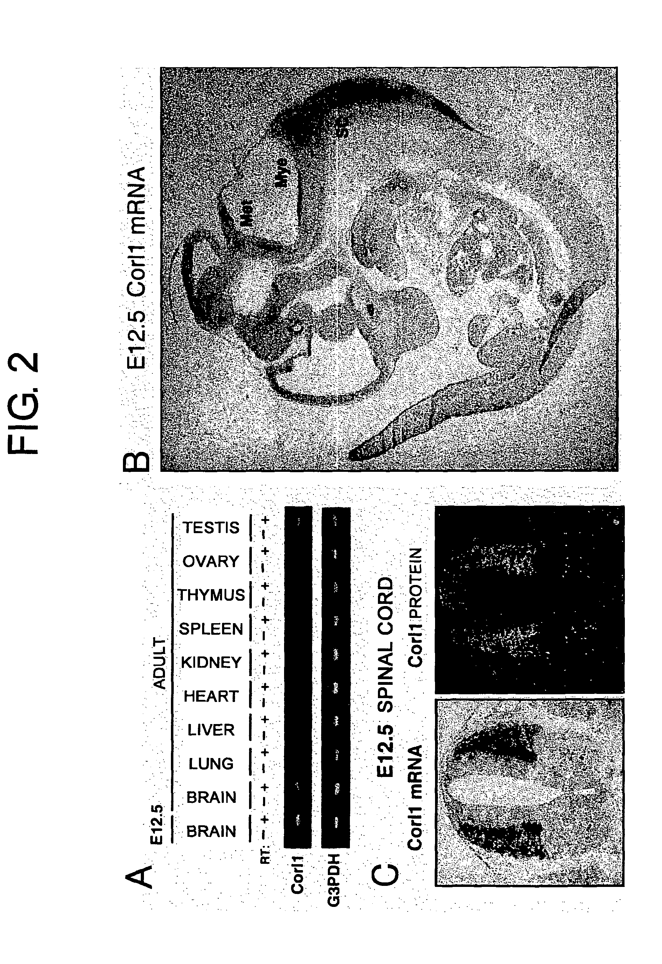

The results revealed that Corl1 was expressed specifically in adult brain and testis (Fig. 2). It was also revealed that the expression level in the brain was higher in fetus than the adult.

Then, the expression was analyzed by in *situ* hybridization using Corl1 gene according to the protocol described below.

First, day 12.5 mouse embryos were embedded in OCT, and a 16 µm fresh cryosections were prepared. The sections were dried on glass slides, and then fixed using 4% PFA at room temperature for 30 minutes. After washing with PBS, hybridization (1 µg/ml DIG-labeled RNA probe, 50% formamide, 5x SSC, 1% SDS, 50 µg/ml yeast RNA, and 50 µg/ml Heparin) was carried out at 65°C for 40 hours. Then, the sections were washed (50% formamide/5x SSC/1% SDS) at 65°C, and treated with RNase (5µg/ml RNase) at room temperature for 5 minutes. The sections were washed with 0.2x SSC at 65°C, and then with 1x TBST at room temperature. After washing, blocking (Blocking reagent: Roche) was carried out. The sections were incubated with alkaline phosphatase-conjugated anti-DIG antibody (DAKO). After washing (1x TBST/2 mM Levamisole), the color was developed using NBT/BCIP (DAKO) as the substrate.

Expression analysis using *in situ* hybridization revealed that Corl1 expression was specific to the central nervous system at E12.5 and it was expressed selectively in some cells of the afterbrain and spinal cord (Fig. 2). Furthermore, it was also revealed that the expression of Corl1 was confined in the dorsal region of spinal cord at developmental stages when the expression was analyzed using transverse sections of the spinal cord. The results described above revealed that Corl1 was selectively expressed in a group of prenatal neurons in the central nervous system.

The expression of Corl1 protein was then analyzed. Anti-Corl1 polyclonal antibody was prepared by the method as described below and expression of Corl1 protein in E12.5 spinal cord was examined.

First, an expression vector was constructed to express a fusion protein between GST and the region of 569 to 813 amino acids of Corl1 which serves as an antigen required for immunization. After this vector was introduced into cells of *E*. *coli* (JM109 strain), expression was induced using IPTG. and the fusion protein was collected using glutathione beads. Rabbits were immunized with the fusion protein several times and the blood was collected. Anti-Corl1 polyclonal antibody was obtained from the sera by affinity purification using the same GST-Corl 1 used as the immunization antigen.

Immunostaining was carried out according to the protocol as described below. E12.5 fetal mice were isolated and fixed with 4% PFA/PBS(-) at 4°C for 7 hours. The solution was replaced with 10% sucrose/PBS(-) at 4°C for 8 hours and then with 20% sucrose/PBS(-) at 4°C overnight and then embedded in OCT. A 12 µm thick section was made. The section were placed onto a glass slide, and then dried at room temperature for 1 hour, were wetted using 0.1 % Triton X-100/PBS(-) for 5 minutes, and then with PBS(-) for 5 minutes. Then, blocking (25% BlockAce/PBS(-)) was carried out at room temperature for 30 minutes. After 1 hour of incubation with a primary antibody at room temperature, the reaction was continues at 4°C overnight. Then, the section was washed four times with 0.1% Triton X-100/PBS(-) at room temperature for 10 minutes. Then, the section was made to react with fluorescently labeled secondary antibody at room temperature for 20 minutes. After washing in the same way as described above, the section was washed twice with PBS(-) at room temperature for 10 minutes and the slide was then mounted. The fluorescence signal was detected under a confocal microscope.

Immunostaining with the anti-Corl1 antibody showed that Corl1 was localized in the nucleus. The expression pattern was the same as that of obtained by *in situ* hybridization. It was thus found that not only mRNA but also protein of Corl1 was expressed in E12.5 spinal cord and furthermore, its signal in *in situ* hybridization and immunostaining were confirmed to be specific (Fig. 2).

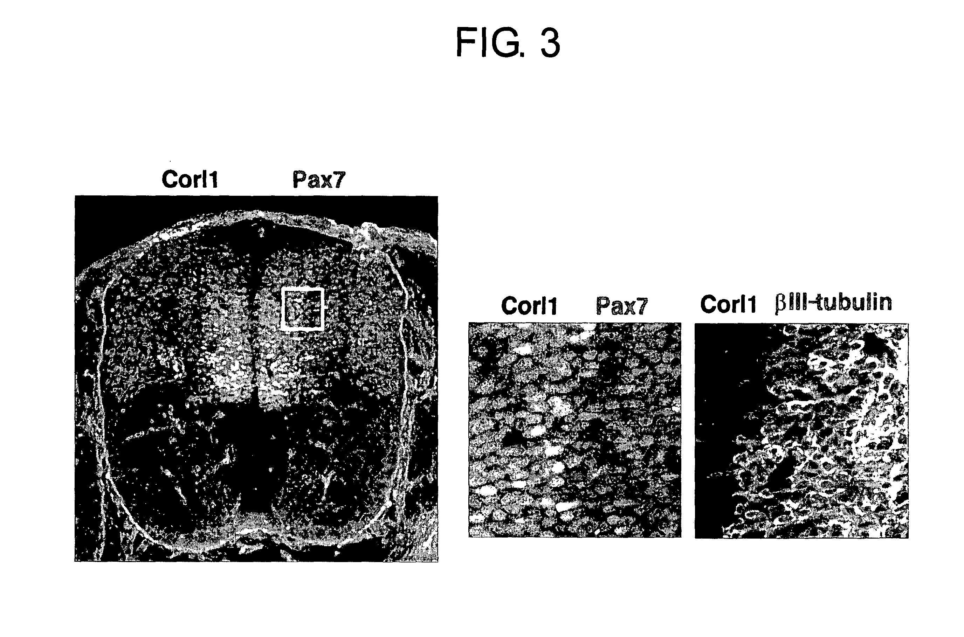

Up to E12.5, neither astrocytes nor oligodendrocytes are developed in the neural tube. Therefore, Corl1 is expected to be expressed in neuronal precursor cells. The expression pattern of neuronal precursor cells at various stages of differentiation was examined. In general, it is known that neurons migrate to the mantle layer (ML) immediately after completion of final division in the ventricular zone (VZ) where proliferating progenitors are present and are matured. First, the expression of Corll was compared with the expression of marker Pax7 for the proliferating progenitors and with the neural precursor marker β-III tubulin to examine whether Corl1 was expressed in the proliferating progenitors or in the neural precursors, which is after the termination of division. The results showed that, Corl1 was expressed only in ML and no coexpression of Corl1 with Pax7 was observed. All Corl1-positive cells expressed β-III tubulin, a marker for precursor cells committed to become neurons. The results described above confirm that Corl1 is specifically expressed in neural precursors after the termination of division (Fig. 3).

The identity of neurons of the spinal cord have been already determined at the stage when β-III tubulin is expressed. Together with the finding that Corl1 was specifically expressed in a group of cells in the spinal cord, this suggest that Corl1 would be useful as a marker to identify types of neurons. The present inventors thus identified the Corl1-expressing cells. It is known that at early developmental stages (E10 to E11.5), 6 types of interneurons, d11 to dI6, are produced at the dorsal region of mouse spinal cord, while at late stages (E12 to E13.5), the same region generates 2 types of interneurons, dILA and dILB. These neurons can be discriminated from one another based on the developmental stage and using selectively expressed markers that are transcription factors. The expression of Corl1 and various markers was thus compared between early (E10.75) and late stages (E 13.25).

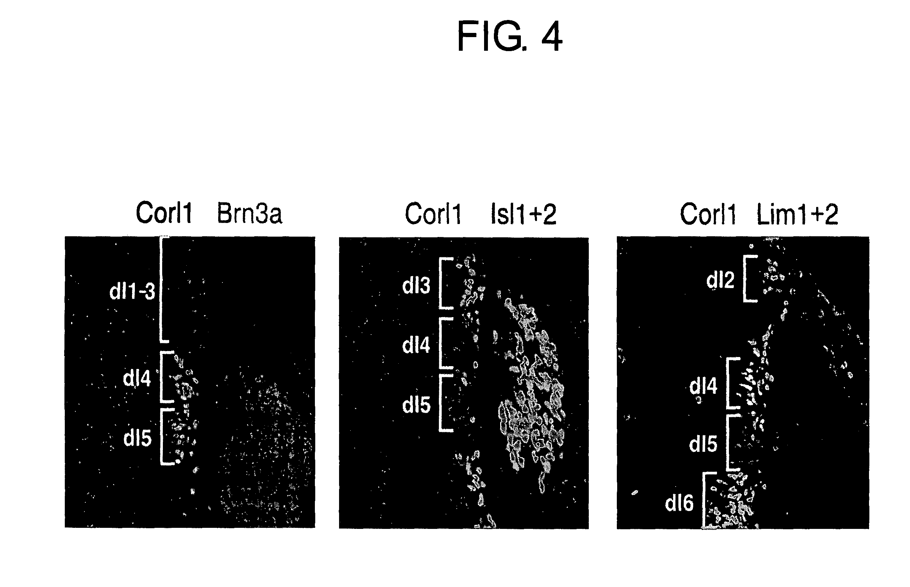

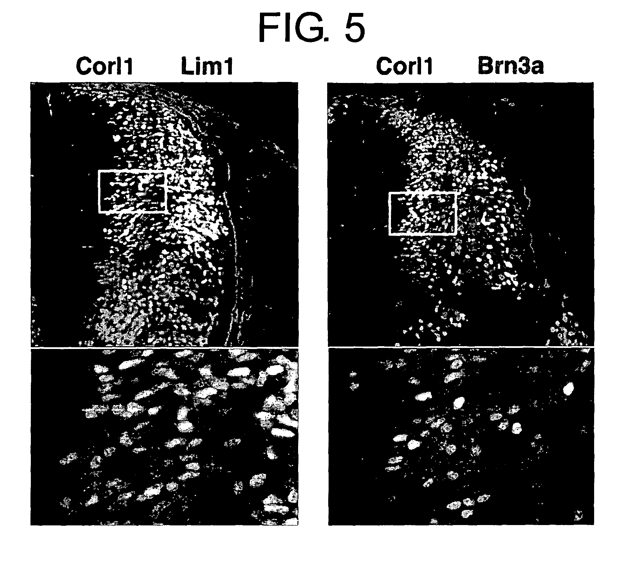

The expression of Corl1 in mouse spinal cord at E10.75 was compared with Brn3a, (a marker for dI1, dI2, dI3, and dI5), Isl1 (a marker for dI3), and Lim1( a marker for dI2, dI4, and dI6). Corl1-positive cells were developed between dI3 and dI6 and because Corl1 was co-expressed in Lim1 for dI4 or Brn3a for dI5, it was thus revealed that Corl1 was specifically expressed in dI4 and dI5 (Fig. 4). Meanwhile, Corl1 was co-expressed in both cells positive in Lim1, a dILA marker, and Brn3, a dILB marker at E13.25. It was thus found that Corl1 was expressed in both dILA and dILB (Fig. 5). The results described above show that Corll is specifically expressed in dI4, dI5, dILA, and dILB and therefore would be useful as a marker for identifying the cell types described above (Fig. 6). In particular, Corl1 appears to be useful as a novel marker for discriminating between dI4 and dI6, which previously could only be differentiated based on developmental location.

### [Example 3] Analysis of Corl1 expression in spinal neurons induced from ES cells in vitro

To examine whether Corl1 can be used to identify *in vitro* differentiated spinal neurons, the expression of Corl1 in spinal neurons induced from ES cells was analyzed according to the following protocol.

CCE cells , an undifferentiated ES cell line, were suspended at a cell density of 1000 cells/10 µl in Glasgow Minimum Essential Medium (Invitrogen) supplemented with 10% Knockout serum replacement (Invitrogen), 2 mM L-glutamine (Invitrogen), 0.1 mM Non-essential amino acid (Invitrogen), 1 mM sodium pyruvate (sigma), 0.1 mM 2-mercaptoethanol (sigma), 100 U/ml penicillin (Invitrogen), and 100 µg/ml streptomycin (Invitrogen). 10 µl of the cells was placed onto the cover of plastic dish. The dish was inverted and incubated at 37°C under 5% CO₂ and 95% humidity for 2 days. Then, formed embryoid bodies (EB) were collected in the above-described medium. 2 µM retinoic acid (RA) (sigma) was added alone or in combination with 300 nM sonic hedgehog (Shh) (R&D) to the medium. The embryoid bodies were further incubated for 5 days, and then washed with PBS-(Sigma). The embryoid bodies were fixed with 4% paraformaldehyde/PBS(-) (Wako) at 4°C for 20 minutes, and washed with PBS(-) (Sigma). Permeablity treatment was given using 0.2% Triton X-100/PBS(-), blocking was achieved using Block-ace (Dainippon Pharmaceutical Co. Ltd). The samples were allowed to react with anti-Corl1 antibody (10 times diluted) and with anti-Lim3 antibody (50 times diluted; Developmental studies hybridoma bank) at room temperature for one hour. The antibody reaction was continued at 4°C overnight. Then, the samples were washed with 0.05% Tween20/PBS(-) and allowed to react with Cy3-labeled anti-rabbit immunoglobulin antibody (10 µg/ml; Jackson) and FITC-labeled anti-mouse immunoglobulin antibody (10 µg/ml; Jackson) at room temperature for one hour. After washing with 0.05% Tween20/PBS(-), the samples were embedded with Prolong (Molecular Probe).

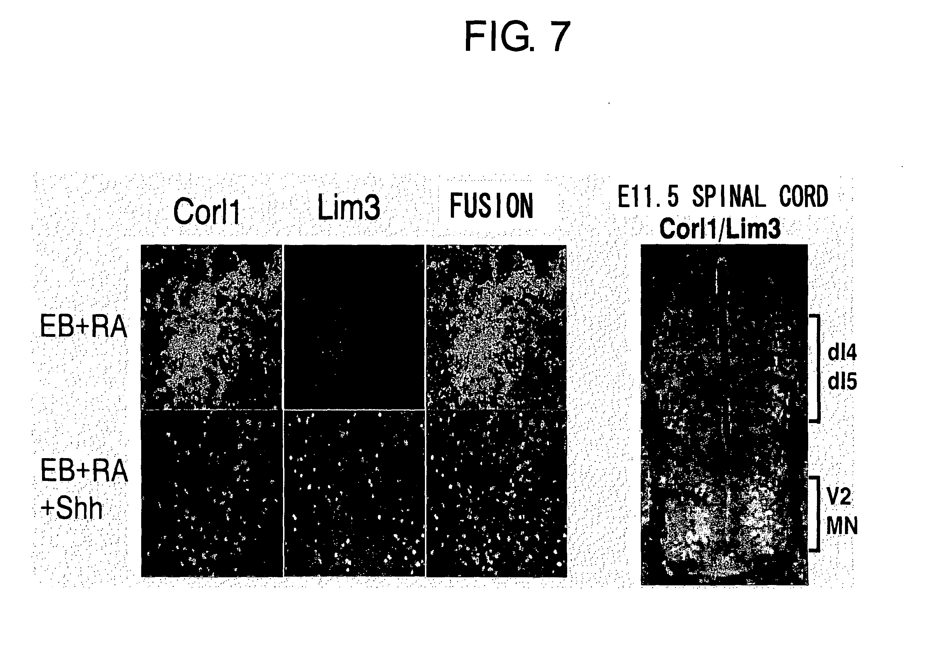

When spinal neurons were induced in the dorsal region in the absence of Shh, Corl1-expressing cells were observed at a high frequency (Fig. 7). Meanwhile, when expressed ventral in the presence of Shh, positive for Lim3 (a marker for motor neuron (MN) and v2 interneuron) appeared. On the contrary, Corll-positive cells decreased. The above described results reveal that even in spinal neurons differentiated from ES cells *in vitro,* Corl1 is expressed depending on the types of cells and thus Corl1 can be used as a marker for identifying cell types.

According to the following protocol, the next step examined whether Corl1 was expressed in dI4 and dI5 in spinal neurons differentiated from ES cells in a similar manner to the *in vivo* expression of Corl1.

Differentiation was induced using the same method as previously described. Then, the samples were fixed with 4% PFA/PBS(-) at 4°C for 2 hours. The solution was replaced with 10% sucrose/PBS(-) at 4°C overnight, and then with 20% sucrose/PBS(-) at 4°C for 6 hours. The samples were embedded in OCT. A 12 µm section was given which was placed onto glass slides, and dried at room temperature for 1 hour. The section was wetted using 0.1% Triton X-100/PBS(-) for 5 minutes, and then with PBS(-) for 5 minutes. Then, blocking (25% BlockAce/PBS(-)) was carried out at room temperature for 30 minutes. After allowing the slide to react with a primary antibody for 1 hour, the reaction was continues at 4°C overnight. Then, the section was washed four times with 0.1% Triton X-100/PBS(-) at room temperature for 10 minutes. Then, the slide was allowed to react with fluorescent labeled secondary antibody at room temperature for 20 minutes. After washing in the same way as described above, the section was washed twice with PBS(-) at room temperature for 10 minutes. The slide was then mounted. The fluorescence signal was detected under a confocal microscope.

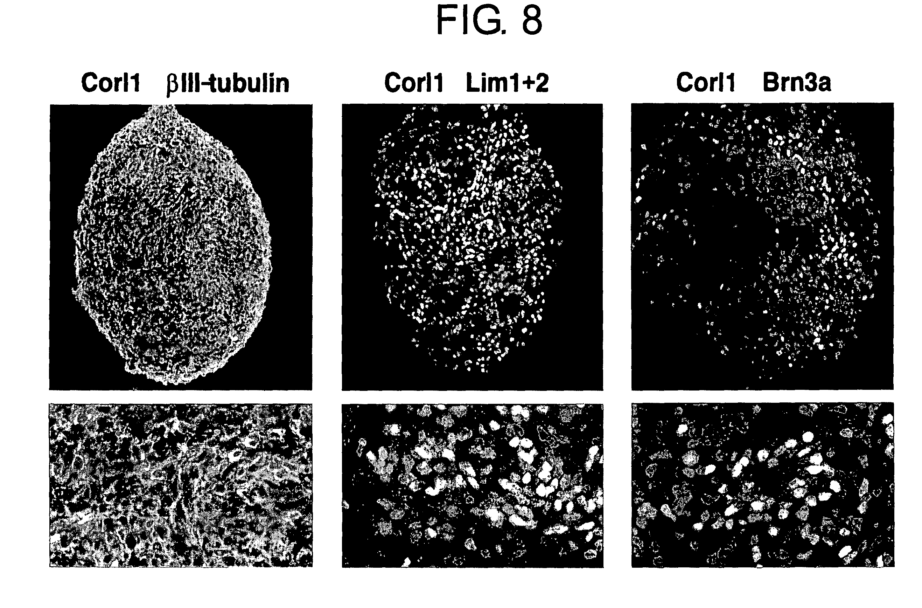

Co-positive Corl1 and Lim1 and co-positive Corl1 and Bm3 were observed (Fig. 8). Corl1 was found to be also expressed in spinal neurons differentiated from ES cells *in vitro* in the same manner as observed in the neurons of mouse embryonic spinal cord.

### Industrial Applicability

In the present invention, the Corl1 gene was identified as specifically expressed in spinal cord interneurons dI4, dI5, dILA, and dILB. Both in terms of safety and therapeutic effect in regeneration medicine for spinal cord injury and the like, it is important to accurately identify the type(s) of neurons regenerated in tissues or neurons induced *in vitro* as a material for transplantation. Corll achieves this objective as a marker for identifying cell types. In particular, cells such as dI4 and dI6 that previously could only be discriminated based on developmental location, i.e., neurons that would differentiate at a random position when induced *in vitro* and thus were indiscriminable *in vitro,* can be distinguished with Corl1.

## Claims

1. A reagent for identifying types of spinal neurons, said reagent comprising as an active component a polynucleotide that hybridizes to a transcript of a Corl1 gene.

2. A reagent for identifying types of spinal neurons, said reagent comprising as an active component a polynucleotide that hybridizes under stringent conditions to at least one polynucleotide having a nucleotide sequence selected from the group consisting of SEQ ID NOs: 1, 3, and 5.

3. A reagent for identifying types of spinal neurons, said reagent comprising as an active component an antibody that binds to a translation product of a Cor1 1 gene.

4. A reagent for identifying types of spinal neurons, comprising as an active component an antibody that binds to at least one polypeptide having an amino acid sequence selected from the group consisting of SEQ ID NOs: 2, 4, and 6, or a partial sequence thereof.

5. A reagent of any one of claims 1 to 4, wherein the target spinal neuron to be identified is dI1, dI2, dI3, dI4, dI5, dI6, dILA, or dILB.

6. A kit for identifying types of spinal neurons, said kit comprising one or more polynucleotides that hybridize to a transcript of a Corl1 gene in combination with one or more polynucleotides that hybridize to a transcript of one or more genes selected from the group consisting of Brn3a, Pax2, Lbx1, Lim1, Lim2, LH2A, LH2B, Isl1, Lmx1b, Tlx1, and Tlx3.

7. A kit for identifying types of spinal neurons, said kit comprising as an active components a polynucleotide that hybridizes under stringent conditions to at least one polynucleotide having a nucleotide sequence selected from the group consisting of SEQ ID NOs: 1, 3, and 5 and a polynucleotide that hybridizes under stringent condition to a transcript of at least one gene selected from the group consisting of Brn3a, Pax2, Lbx1, Lim1, Lim2, LH2A, LH2B, Isl1, Lmx1b, Tlx1, and Tlx3.

8. A kit for identifying types of spinal neurons, said kit comprising an antibody that binds to a translation product of a Corl1 gene in combination with an antibody that binds to a translation product of one or more genes selected from the group consisting of Brn3a, Pax2, Lbx1, Lim1, Lim2, LH2A, LH2B, Isl1, Lmx1b, Tlx1, and Tlx3.

9. A kit for identifying types of spinal neurons, said kit comprising as active components an antibody that binds to at least one polypeptide having an amino acid sequence selected from the group comprising SEQ ID NOs: 2, 4, and 6, or a partial sequence thereof and an antibody that binds to a translation product of at least one gene is selected from the group consisting of Brn3a, Pax2, Lbx1, Lim1, Lim2, LH2A, LH2B, Isll, Lmx1b, Tlx1, and Tlx3.

10. A kit of any one of claims 6 to 9, wherein the target spinal neuron to be identified is dI1, dI2, dI3, dI4, dI5, dI6, dILA, or dILB.

11. A method for identifying types of spinal neurons, said method comprising the step of detecting a transcript or translation product of a Corl1 gene in spinal neurons.

12. The method of claim 11, said method comprising the step of detecting a transcript or translation product of one or more genes selected from the group consisting of Brn3a, Pax2, Lbx1, Lim1, Lim2, LH2A, LH2B, Isl1, Lmx1b, Tlx1, and Tlx3.

13. The method of claim 11 or 12, wherein the target spinal nerve cell to be identified is dI1, dI2, dI3, dI4, dI5, dI6, dILA, or dILB.

14. A method for identifying types of spinal neurons, comprising the steps of contacting spinal neurons with:
(1) a polynucleotide that hybridizes under stringent conditions to at least one polynucleotide having a nucleotide sequence selected from the group consisting of SEQ ID NOs: 1, 3, and 5; or
(2) an antibody that binds to a polypeptide comprising an amino acid sequence selected from the group consisting of SEQ ID NOs: 2, 4, and 6, or a partial sequence thereof.

15. The method of claim 14, comprising the steps of contacting spinal neurons with:
(1) a polynucleotide which hybridizes under stringent conditions to a transcript of at least one gene selected from the group consisting of Brn3a, Pax2, Lbx1, Lim1, Lim2, LH2A, LH2B, Isl1, Lmx1b, Tlx1, and Tlx3; or
(2) an antibody that binds to a translation product of at least one gene selected from the group consisting of Brn3a, Pax2, Lbx1, Lim1, Lim2, LH2A, LH2B, Isl1, Lmx1b, Tlx1, and Tlx3.

16. The method of claim 14 or 15, further comprising the step of discriminating the group consisting of at least one spinal neuron type selected from dI1, dI2, dI3, and dI6 and the group consisting of at least one spinal neuron type selected from dI4, dI5, dILA, and dILB.

17. The method of any one of claims 14 to 16, said method comprising the step of discriminating between a spinal neuron type that expresses a transcript of one or more genes selected from the group consisting of Brn3a, Pax2, Lbx1, Lim1, Lim2, LH2A, LH2B, Isl1, Lmx1b, Tlx1, and Tlx3 and a spinal neuron type that does not express a transcript of one or more genes selected from the group consisting of Brn3a, Pax2, Lbx1, Lim1, Lim2, LH2A, LH2B, Isl1, Lmx1b, Tlx1, and Tlx3.

18. A method for screening for compounds that induce differentiation of cells that have a potential to differentiate into spinal neurons, said method comprising the steps of:
(a) inducing differentiation of cells that have a potential to differentiate into spinal neuron in the presence of a test sample;
(b) detecting a transcript or translation product of a Corl1 gene in the differentiated cells; and
(c) selecting a test sample that increases the level of the Corl transcript or translation product as compared with the level determined in the absence of the test sample.

19. The method of claim 18, wherein the cells that have a potential to differentiate into spinal neuron are ES cells.

20. The use of:
(a) a polynucleotide that hybridizes to a transcript of a Corl1 gene; or
(b) an antibody which binds to a translation product of a Corl1 gene;
in the production of reagents for identifying types of spinal neurons.
